(19) Europäisches Patentamt / European Patent Office / Office européen des brevets

(11) **EP 4 699 582 A1**

(12) **EUROPEAN PATENT APPLICATION**
published in accordance with Art. 153(4) EPC

(43) Date of publication:
**25.02.2026 Bulletin 2026/09**

(21) Application number: **23934202.5**

(22) Date of filing: **28.07.2023**

(51) International Patent Classification (IPC):
*A61F 5/02* (2006.01)   *A61F 5/30* (2006.01)

(86) International application number:
**PCT/KR2023/011090**

(87) International publication number:
**WO 2024/219555 (24.10.2024 Gazette 2024/43)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:   **20.04.2023   KR 20230051866**

(71) Applicants:
• **Seoul National University R&DB Foundation**
  **Seoul 08826 (KR)**
• **Seoul National University Hospital**
  **Seoul 03080 (KR)**

(72) Inventors:
• **PARK, Jae Heung**
  **Seoul 08826 (KR)**
• **KIM, Joo Wan**
  **Seoul 08826 (KR)**
• **CHUNG, Sun Gun**
  **Seoul 02839 (KR)**
• **KIM, Kee Won**
  **Gwacheon-si Gyeonggi-do 13836 (KR)**
• **JO, Woo Sup**
  **Seoul 06981 (KR)**

(74) Representative: **TRBL Intellectual Property**
**Plaza de Castilla 3, 7°A**
**28046 Madrid (ES)**

(54) **DYNAMIC BELT-TYPE BRACE**

(57)   The present invention relates to a dynamic belt-type brace, specifically to an apparatus that dynamically adjusts abdominal pressure, and more specifically to an apparatus that dynamically adjusts abdominal pressure to lighten the load on the muscles around the vertebra and reduce the shearing and pressing force exerted on the vertebral body.

**Fig. 1.**

EP 4 699 582 A1

## Description

**[Technical Field]**

The present invention relates to a belt-type dynamic orthosis, and specifically to a device for dynamically adjusting intra-abdominal pressure; more specifically, to a belt-type device capable of dynamically adjusting intra-abdominal pressure to reduce the burden on paraspinal muscles and to decrease shear and compressive forces of the vertebral body.

**[Background Art]**

According to the National Health Insurance Review and Assessment Service's statistics on diseases of public interest, the number of patients who visited hospitals in Korea for spinal disorders exceeded 9 million as of 2019, and has been steadily increasing by 2-3% each year. In addition, spinal disorders are common diseases that 80% of the Korean population experience during their lifetime, and even among younger people in their 20s and 30s (the so-called 2030 generation), the incidence has also been steadily increasing in recent years.

In particular, the lumbar spine is the region that bears the greatest load in the spine and, at the same time, contributes the most to spinal motion. For this reason, spinal disorders mainly occur in the lumbar spine, and a prescription to use a spinal orthosis is recommended as a conservative treatment therefor.

A spinal orthosis is a device used for the treatment and prevention of patients with spinal disorders (low back pain, vertebral compression fracture, spinal instability, after spinal surgery, etc.), which supports the spine, provides stability, and distributes the pressure applied to the spine. Various types of spinal orthoses exist, and the purpose of use and type are determined according to the individual circumstances of the patient; the most common spinal orthosis is a belt-type spinal orthosis configured to wrap the waist region to support the spine while reducing spinal flexion.

**In** general, a belt-type spinal orthosis is worn to wrap the waist or abdomen and is used to fix the spine by compressing the waist or abdomen, and various forms of belts having functions beneficial to health are commercially available. In a conventional belt-type spinal orthosis, the belt is wound around the waist to wrap the wearer's waist or abdomen, and the two ends are coupled by a coupling means such as hook-and-loop fasteners or male-female buckles to tighten the waist or abdomen strongly, thereby supporting the waist and abdomen. By compressing the waist, intra-abdominal pressure is increased and vertical and horizontal pressures between vertebral bodies are reduced, thereby alleviating low back pain and at the same time providing the effect of increasing spinal stability. However, the conventional belt-type spinal orthosis has the limitation that it possesses only simple physical functions such as merely fixing the spine by compressing the waist or preventing protrusion of the abdomen.

As such, currently marketed belt-type spinal orthoses have static characteristics that provide a constant compressive force regardless of the wearer's posture or intra-abdominal pressure. Due to these static characteristics, long-term wear induces atrophy of the muscles around the waist, causing adverse effects that may worsen the lumbar condition; it may also produce psychological dependence on the belt-type spinal orthosis as the spinal muscles and ligaments become weakened, and, because the spine is uniformly compressed and supported irrespective of spinal movement, the wearer experiences discomfort.

Moreover, when the wearer makes sudden movements such as coughing or bending the waist to lift a heavy object, the abdomen may be instantaneously constricted due to a belt whose length remains invariable. At this time, the wearer not only feels discomfort and pain due to the momentary compressive force, but in severe cases even blood pressure may rise, potentially causing health problems.

In addition, such spinal orthoses, as more functions are mounted, have been released for industrial assistive use exhibiting stiffness and have been implemented in forms akin to wearable robots; many robots were connected to the hip (gluteal) side and assisted only a single large joint, and there has been no robot that targets the lumbar region to apply intra-abdominal pressure to the waist by performing active, precise control according to posture.

**[Prior Art Documents]**

**[Patent Document]**

(Patent Document 1) Korean Registered Patent No. 10-1664351

**[Problems to be Solved]**

The present invention is intended to propose a belt-type robot which, by using an actuator and a multi-pulley mechanism, can provide dynamic abdominal compression and appropriate support for the spine according to movements of the wearer's lumbar region.

The present invention is to provide an active assistive tool that, when spinal motion occurs, provides proportional abdominal pressure and support according to the range of motion.

Further, the present invention also has the object of providing a mechanism that increases spinal stability while minimizing weakening of muscles around the spine.

In addition, an object of the present invention is to solve the above-described problems of conventional belt-type spinal orthoses, by providing a belt-type dynamic orthosis having dynamic characteristics that complement the static characteristics which are drawbacks of the prior art belt-type spinal orthoses.

More specifically, the object is to provide a belt-type dynamic orthosis that, when movement of the wearer's spine occurs, estimates the wearer's posture and intra-abdominal pressure, and provides appropriate abdominal compression and support for the spine in response to the estimated posture and intra-abdominal pressure.

The objects of the present invention are not limited to those mentioned above, and other objects not mentioned will be clearly understood by those skilled in the art from the description below.

**[Means for Solving the Problems]**

In order to solve the above-described problems, one aspect of the present invention provides a belt-type dynamic orthosis comprising: a belt body including a back portion that faces the wearer's back, and left and right portions respectively located to the left and right of the back portion and configured to be mutually couplable; a first stretchable portion located between the back portion and the left portion; a second stretchable portion located between the back portion and the right portion; a plurality of first pulleys disposed in the first stretchable portion; a plurality of second pulleys disposed in the second stretchable portion; at least one wire connected to the plurality of first pulleys and the plurality of second pulleys; an actuator disposed in either one of the left portion and the right portion and driven to wind or unwind the at least one wire; at least one load cell connected to any one pulley among the first pulleys or to any one pulley among the second pulleys, the at least one load cell measuring a tension applied to the wire when the at least one wire is pulled and released; and a controller for controlling the actuator.

According to one embodiment, a pressure-tension modeling that represents a relationship between the wearer's intra-abdominal pressure and a tension applied to the wire is stored in the controller, and the controller may be configured to estimate the wearer's intra-abdominal pressure by inputting a tension measured by the load cell into the pre-stored pressure-tension modeling and, based on the estimated intra-abdominal pressure, control the actuator to adjust the tension applied to the wire.

According to one embodiment, the belt-type dynamic orthosis further includes two inertial measurement sensors respectively disposed at a back region and a waist region, a pressure profile according to the wearer's posture is stored in the controller, and the controller may calculate a relative angle of the two inertial measurement sensors to estimate the wearer's posture and control the actuator to adjust the tension applied to the wire such that the estimated intra-abdominal pressure dynamically follows the pressure profile according to the estimated posture of the wearer.

According to one embodiment, the back region and the waist region may be positions corresponding to the wearer's fifth thoracic vertebra (T5) and second sacral vertebra (S2), respectively.

According to one embodiment, the load cell may be connected to any one pulley among the plurality of first pulleys or to any one pulley among the plurality of second pulleys.

According to one embodiment, the plurality of first pulleys may be alternately disposed on a back-side fixed portion of the first stretchable portion and a left-side fixed portion of the first stretchable portion, and the plurality of second pulleys may be alternately disposed on a back-side fixed portion of the second stretchable portion and a right-side fixed portion of the second stretchable portion.

According to one embodiment, the plurality of first pulleys and the plurality of second pulleys are each an even number, the at least one wire includes a first wire connected to the plurality of first pulleys and a second wire connected to the plurality of second pulleys, one end of the first wire is connected to the actuator and the other end is connected to the back-side fixed portion of the first stretchable portion, and one end of the second wire is connected to the actuator and the other end is fixed to the back-side fixed portion of the second stretchable portion.

According to one embodiment, the plurality of first pulleys and the plurality of second pulleys are each an odd number, the at least one wire includes a first wire connected to the plurality of first pulleys and a second wire connected to the plurality of second pulleys, one end of the first wire is connected to the actuator and the other end is fixed to the left-side fixed portion of the first stretchable portion, and one end of the second wire is connected to the actuator and the other end is fixed to the right-side fixed portion of the second stretchable portion.

According to one embodiment, the at least one wire includes only one wire, pulleys among the plurality of first pulleys and the plurality of second pulleys that are adjacent to the actuator are configured as an even number, pulleys among the plurality of first pulleys and the plurality of second pulleys that are distant from the actuator are configured as an odd number, and the single wire may be configured to form a closed loop by being connected from the actuator to the plurality of first pulleys, to the back portion, and to the plurality of second pulleys, and then, via the back portion again, connected to the actuator.

According to one embodiment, the first stretchable portion is positioned to face the wearer's left flank, and the second stretchable portion may be positioned to face the wearer's right flank.

According to one embodiment, the actuator may include a brushless DC motor, a worm gear, and a wire pulley.

According to one embodiment, the actuator may be a pneumatic actuator or a hydraulic actuator.

**[Effects of the Invention]**

According to the configuration of the present invention described above, by dynamically responding to changes in posture and intra-abdominal pressure according to the wearer's movements and providing appropriate abdominal compression and support for the spine, it is possible to obtain markedly improved spinal stability with fewer side effects compared to conventional belt-type spinal orthoses, while at the same time providing greater comfort to the wearer.

According to embodiments of the present invention, there is provided a spinal orthosis that provides stability in proportion to the mechanical support required according to the wearer's spinal movements, whereby the effects of existing spinal orthoses are sufficiently maintained while overcoming side effects in which continuous spinal support weakens muscles around the spine.

**[Brief Description of the Drawings]**

FIG. 1 is a diagram schematically showing a belt-type dynamic orthosis according to one aspect of the present invention.

FIG. 2 is a diagram showing an exemplary arrangement of a plurality of first pulleys disposed in the first stretchable portion.

FIG. 3 is a diagram obtained by enlarging a dotted rectangular portion of FIG. 1, and shows in detail a pulley and a load cell connected thereto.

FIG. 4 schematically shows a rear view when a wearer wears the belt-type dynamic orthosis according to one aspect of the present invention.

FIG. 5 shows a pressure measurement value according to an intra-abdominal pressure estimation technique mounted on the belt-type dynamic orthosis according to one aspect of the present invention and a comparative pressure value of a commercial pressure sensor (blue: proposed device, red: commercial sensor).

FIG. 6 is a graph showing, in an experiment on five male subjects, results of measuring muscle activity during a flexion section where a 5 kg load box is lifted with the waist bent and an extension section where the waist is straightened.

FIG. 7 is a graph showing, in an experiment on five male subjects, results of measuring linear acceleration in a fore-aft swaying direction of the torso to confirm the effect of spinal stability.

**[Detailed Description for Carrying Out the Invention]**

The embodiments of the present invention are illustrated for the purpose of explaining the technical idea of the present invention. The scope of rights according to the present invention is not limited to the embodiments presented below or to the specific descriptions of these embodiments.

Unless otherwise defined, all technical and scientific terms used in the present invention have meanings generally understood by those of ordinary skill in the art to which the present invention pertains. All terms used in the present invention are selected for the purpose of more clearly describing the present invention, and are not selected to limit the scope of rights according to the present invention.

Expressions such as "including," "comprising," and "having" used in the present invention are to be understood, unless otherwise mentioned in the phrase or sentence in which the expression appears, as open-ended terms that imply the possibility of including other embodiments.

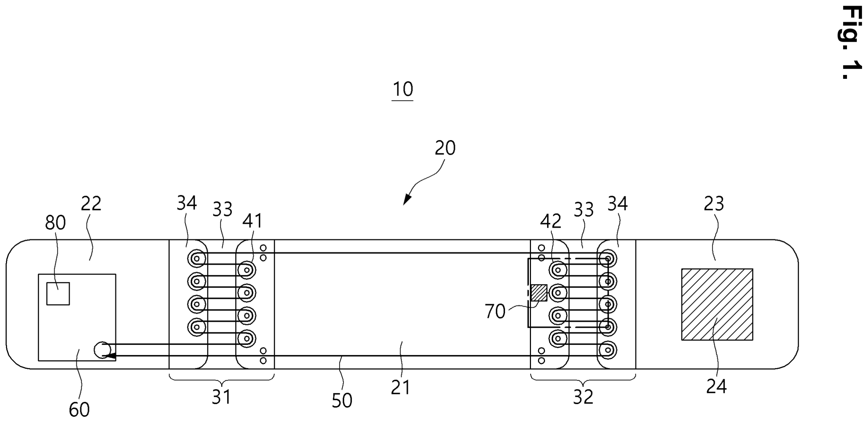

In the present invention, based on the embodiment shown in FIG. 1, the left-right direction is defined as the "longitudinal direction," and the up-down direction is defined as the "width direction." In addition, based on the embodiment shown in FIG. 1, the left side of the drawing is defined as the left side, and the right side of the drawing is defined as the right side.

Singular expressions described in the present invention may include plural meanings unless otherwise mentioned, and the same applies to singular expressions recited in the claims.

Hereinafter, embodiments of the present invention will be described with reference to the accompanying drawings. In this process, the thicknesses of lines or the sizes of components shown in the drawings may be depicted exaggeratedly for the sake of clarity and convenience of description. In addition, in the following description of embodiments, descriptions of the same or corresponding components may be omitted to avoid redundancy. However, even if the description of a component is omitted, it is not intended that such a component is not included in an embodiment.

Furthermore, the embodiments below do not limit the scope of rights of the present invention, but are merely exemplary matters of the components presented in the claims of the present invention; embodiments that are included in the technical idea throughout the specification of the present invention and include components that can be substituted as equivalents for the components in the claims may fall within the scope of rights of the present invention.

**Belt-Type Dynamic Orthosis**

In general, belts that dynamically compress the abdomen are also medically referred to as "compression belts" or "compression bandages," and are used for purposes such as supporting damaged organs or muscles or suppressing bleeding. However, the belt-type dynamic orthosis proposed in the present invention relates to a belt-type orthosis used for purposes such as supporting and protecting weakened muscles in order to alleviate pain in the abdominal or spinal region, increase spinal stability, and help maintain correct posture.

Such belt-type orthoses may be used, by way of example, for purposes of providing stability to the spine, correcting the spine, protecting muscles, alleviating pain, and improving motor function.

In the present invention, the term "dynamic orthosis" may mean an orthosis that is "actively" driven, and may be understood as moving in response to an external input or operation. In one embodiment, the belt-type dynamic orthosis of the present invention may be dynamically driven in accordance with intra-abdominal pressure according to the wearer's posture.

First, with reference to FIG. 1, a belt-type dynamic orthosis (10) according to one aspect of the present invention will be described in detail.

As shown in FIG. 1, the belt-type dynamic orthosis (10) according to one aspect of the present invention is configured to include: a belt body (20) including a back portion (21) that faces the wearer's back, and left and right portions (22, 23) respectively located to the left and right of the back portion (21) and configured to be mutually couplable; a first stretchable portion (31) located between the back portion (21) and the left portion (22); a second stretchable portion (32) located between the back portion (21) and the right portion (23); a plurality of first pulleys (41) disposed in the first stretchable portion (31); a plurality of second pulleys (42) disposed in the second stretchable portion (32); at least one wire (50) connected to the plurality of first pulleys (41) and the plurality of second pulleys (42); an actuator (60) disposed in either one of the left portion (22) and the right portion (23) and driven to wind or unwind the at least one wire (50); at least one load cell (70) connected to any one pulley among the first pulley (41) or the second pulley (42) and configured to measure a tension applied to the wire (50) when the at least one wire (50) is pulled and released; and a controller (80) for controlling the actuator (60).

FIG. 1 is a diagram schematically showing one embodiment of the belt-type dynamic orthosis (10) according to one aspect of the present invention. As shown in FIG. 1, the belt-type dynamic orthosis (10) according to one aspect of the present invention may include the belt body (20) including the back portion (21), the left portion (22), and the right portion (23), and first and second stretchable portions (31, 32) including an elastic material or structure. The first stretchable portion (31) may be located between the left portion (22) and the back portion (21), and the second stretchable portion (32) may be located between the right portion (23) and the back portion (21). That is, as shown in FIG. 1, the belt body (20) may be configured, in order from the left side of the drawing, of the left portion (22), the first stretchable portion (31), the back portion (21), the second stretchable portion (32), and the right portion (23).

Among the components of the belt body (20), the back portion (21) is a portion that faces a part of the wearer's back and/or waist when the belt-type dynamic orthosis (10) according to one aspect of the present invention is worn, and, as shown in FIG. 1, includes one or more paths through which the wire (50) passes. Among the components of the belt body (20), the left portion (22) and the right portion (23) are portions that face the wearer's abdominal region when the belt-type dynamic orthosis (10) according to one aspect of the present invention is worn, and include engagement means (24), preferably of a male-female type, for enabling the left portion (22) and the right portion (23) to be couplable, and an actuator driven to wind or unwind the at least one wire (50).

In the present invention, the material of the belt body (20) is not particularly limited, but in comprehensive consideration of weight, breathability, and durability, it is preferable that it be made of a mesh material. In addition, dimensions such as the size and height of the belt body (20) are not particularly limited, and it is preferable that it be manufactured to match body measurements such as the wearer's height, weight, and abdominal circumference.

When a wearer intends to wear the belt-type dynamic orthosis (10) according to one aspect of the present invention, the wearer first aligns the back portion (21) with the wearer's back or waist, then wraps the left portion (22) and the first stretchable portion (31) around the left flank, wraps the right portion (23) and the second stretchable portion (32) around the right flank to encircle the waist as a whole, and then fastens the engagement means (24) provided on the right portion (23) and the left portion (22) to complete wearing. A wearer whose both arms are free may wear it on the waist by himself or herself, and, in the case of a person with a disability in both arms or in one arm, another person may fasten it around the waist.

The wearer may complete wearing by fastening the engagement means (24) provided on the right portion (23) and the left portion (22), and the engagement means (24) itself may freely employ conventional techniques. In a non-limiting embodiment, the left portion (22) and the right portion (23) may be coupled by Velcro, in which case a hook and a loop of Velcro may be stitched and provided respectively on the rear surface of the left portion (22) and the front surface of the right portion (23), or on the front surface of the left portion (22) and the rear surface of the right portion (23). For example, as shown in FIG. 1, hooks and loops are provided on the rear surface of the left portion (22) and the front surface of the right portion (23) so that the wearer can easily wear the belt-type dynamic orthosis (10) by overlapping the left portion (22) and the right portion (23) and attaching the hooks and loops of the left portion (22) and the right portion (23) facing each other.

In another non-limiting embodiment, at least one female buckle and at least one male buckle may be provided at the distal ends of the left portion (22) and the right portion (23), respectively, and these female and male buckles may use buckles that are generally installed on belts or bags and used for fastening.

The belt-type dynamic orthosis (10) according to one aspect of the present invention includes the first stretchable portion (31) located between the back portion (21) and the left portion (22) and the second stretchable portion (32) located between the back portion (21) and the right portion (23). When worn, the first stretchable portion (31) may be positioned to face the wearer's left flank, and the second stretchable portion (32) may be positioned to face the wearer's right flank. The first and second stretchable portions (31, 32) may be formed of an elastic material and may include a stretchable portion (33) configured such that its length is adjustable in the longitudinal direction of the belt body (20), and fixed portions (34) provided on both sides in the longitudinal direction of the stretchable portion (33), which are made of a relatively tough and rigid material so that a plurality of pulleys (41, 42) can be fixed thereto. Preferably, as shown in FIG. 1, the stretchable portion (33) is provided over the entire length of each of the first and second stretchable portions (31, 32), and the fixed portions (34) may be provided so as to at least partially overlap the stretchable portion (33) above and on both sides in the longitudinal direction.

The elastic material constituting the stretchable portion (33) of the first stretchable portion (31) and the second stretchable portion (32) is not particularly limited, and any structure or material exhibiting viscosity or elasticity may be applied without limitation. For example, a spring, an elastic band, a clutch, or a brake may be applied. It is preferable that the fixed portions (34) of the first stretchable portion (31) and the second stretchable portion (32) be manufactured more rigidly than other portions of the belt body (20) so that a plurality of pulleys can be firmly fixed. To this end, additional stitching may be added, or a different, more durable material may be applied only to these fixed portions (34). The plurality of pulleys may be directly attached to the fixed portions (34), or may be connected at a predetermined distance by using a strap or separate connecting components; however, in consideration of durability and stability of connection of the wire (50), it is preferable that they be directly attached to the fixed portions (34).

## Multi-Pulley Mechanism

The belt-type dynamic orthosis (10) according to one aspect of the present invention includes a plurality of pulleys in each of the first stretchable portion (31) and the second stretchable portion (32), and these plural pulleys include a multi-pulley mechanism (40) for amplifying a driving force of the wire (50). For convenience of description in the present invention, the plurality of pulleys disposed in the first stretchable portion (31) are defined as first pulleys (41), and the plurality of pulleys disposed in the second stretchable portion (32) are defined as second pulleys (42). Here, a "pulley" refers to a sheave wheel used to transmit force or change direction by winding a rope or wire therearound; the pulley itself may employ conventional techniques, and a detailed description and illustration of the pulley configuration will be omitted.

It is preferable that the plurality of pulleys provided in the first stretchable portion (31) and the second stretchable portion (32) be arranged to be bilaterally symmetrical with the back portion (21) as a center. For example, it is preferable that the arrangement of the plurality of first pulleys (41) disposed in the first stretchable portion (31) and the arrangement of the plurality of second pulleys (42) disposed in the second stretchable portion (32) form mirror symmetry with respect to a center line in the width direction of the back portion (21). Through the symmetric structure, there is an effect that the sense of resistance applied to the user when bending or extending is reduced.

By applying a multi-pulley system in the present invention, the wearer can enjoy better wearing comfort due to uniform pressure distribution, various locomotor activity can be provided by allowing the spine to move freely, and adaptability according to various spinal environments of users can be implemented.

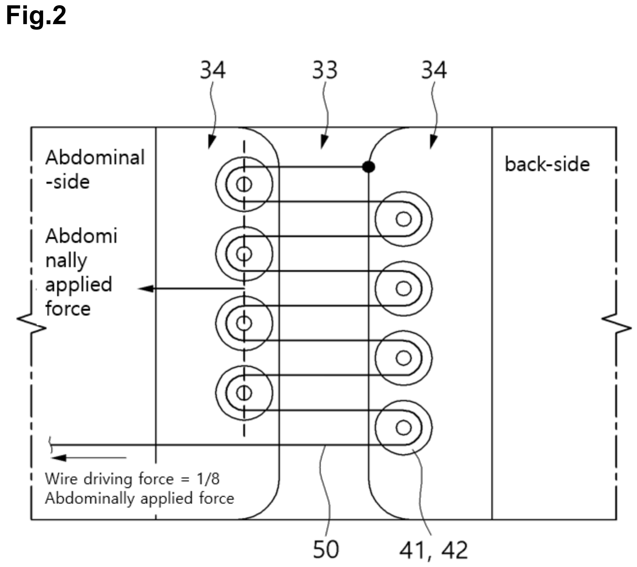

The first pulleys (41) disposed in the first stretchable portion (31) are provided in plurality, and the plurality of first pulleys (41) may be alternately arranged on a back-portion (21) side fixed portion (34) of the first stretchable portion (31) and on a left-portion (22) side fixed portion (34) of the first stretchable portion (31). FIG. 2 shows an exemplary arrangement of the plurality of first pulleys (41) disposed in the first stretchable portion (31). With reference to FIG. 2, the plurality of first pulleys (41) are alternately arranged, starting with a first pulley arranged on the back-portion (21) side fixed portion (34) of the first stretchable portion (31), and, while moving upward toward an upper side of the belt body (20) in the width direction, alternately on the left-portion (22) side fixed portion (34) of the first stretchable portion (31) and on the back-portion (21) side fixed portion (34) of the first stretchable portion (31). **In** the embodiment shown in FIG. 2, the plurality of first pulleys (41) consist of eight pulleys in total, and an end of the wire (50) is fixed to a part of the back-portion (21) side fixed portion (34). However, the invention is not limited thereto, and depending on the number of the plurality of pulleys, an end of the wire (50) may be fixed to the left-portion (22) side fixed portion (34) or to the right-portion (23) side fixed portion (34).

The second pulleys (42) disposed in the second stretchable portion (32) are provided in plurality, and the plurality of second pulleys (42) may be alternately arranged on a back-portion (21) side fixed portion (34) of the second stretchable portion (32) and on a right-portion (23) side fixed portion (34) of the second stretchable portion (32). So as to be mirror-symmetric with the exemplary arrangement of the plurality of first pulleys (41) disposed in the first stretchable portion (31) shown in FIG.

2, with respect to the center line in the width direction of the back portion (21), the plurality of second pulleys (42) are alternately arranged, starting with a first pulley arranged on the back-portion (21) side fixed portion (34) of the second stretchable portion (32), and, while moving upward toward an upper side of the belt body in the width direction, alternately on the right-portion (23) side fixed portion (34) of the second stretchable portion (32) and on the back-portion (21) side fixed portion (34) of the second stretchable portion (32).

## Wire

The belt-type dynamic orthosis (10) according to one aspect of the present invention includes at least one wire (50) connected to the plurality of first pulleys (41) or the plurality of second pulleys (42), and having one end connected to the actuator (60).

In the present invention, the material of the wire (50) is not particularly limited, and various materials may be applied without limitation depending on a specific application environment or design. In addition, in the present invention, physical properties such as the length, thickness, and strength of the wire (50) are not particularly limited, and it is preferable to determine the conditions of the wire (50) so that the wire can sufficiently withstand the force applied by the actuator (60).

In a non-limiting embodiment, the at least one wire (50) may include a first wire (51) connected to the plurality of first pulleys (41) and a second wire (52) connected to the plurality of second pulleys (42). In this case, the plurality of first pulleys (41) and the plurality of second pulleys (42) may be configured as an even number or as an odd number. When the plurality of first pulleys (41) and the plurality of second pulleys (42) are configured as an even number, one end of the first wire (51) is connected to the actuator (60) and the other end is fixed to a back-portion (21) side fixed portion (34) of the first stretchable portion (31), and one end of the second wire (52) is connected to the actuator (60) and the other end is fixed to a back-portion (21) side fixed portion (34) of the second stretchable portion (32). Conversely, when the plurality of first pulleys (41) and the plurality of second pulleys (42) are configured as an odd number, one end of the first wire (51) is connected to the actuator (60) and the other end is fixed to a left-portion (22) side fixed portion (34) of the first stretchable portion (31), and one end of the second wire (52) is connected to the actuator (60) and the other end is fixed to a right-portion (23) side fixed portion (34) of the second stretchable portion (32).

FIG. 2 illustrates an exemplary embodiment in which, in the first stretchable portion (31) located on the left side of the back portion (21), the first wire (51) is connected to the plurality of first pulleys (41) formed as an even number. With reference to FIG. 2, the first wire (51) extending from the actuator (60) is first wound around a first pulley fixed to the back-portion (21) side fixed portion (34), then extends toward a second pulley fixed to the left-portion (22) side fixed portion (34) on the opposite side and is wound around the second pulley. Subsequently, after being wound sequentially from a third pulley to an eighth pulley, the other end of the first wire (51) is fixed to a portion of the back-portion (21) side fixed portion (34). When a wire pulley of the actuator winds and pulls the first wire (51), the force pulled by the actuator, i.e., the wire driving force, is amplified by the number of pulleys by the multi-pulley mechanism (40)-eightfold in the embodiment of FIG. 2-and applied to the abdomen. In other words, in the present invention, when driving the wire (50), only a small force equal to the force to be applied to the abdomen divided by the number of pulleys may be required.

Through such a multi-pulley mechanism (40), an actuator of small output relative to the required force can be used, thereby enabling weight reduction of the belt-type dynamic orthosis (10) and lowering manufacturing cost. **In** addition, in the case of existing belts, the adjustment unit is often located at the back portion and uniformly restricts the spine, providing resistance to flexion/extension movements of the lumbar spine; however, in the dynamic orthosis according to the embodiments of the present invention, the adjustment units (multi-pulley mechanisms) are arranged on both sides of the wearer so that flexion/extension movements of the spine can be restricted or released as needed.

Further, by using the multi-pulley mechanism (40) by the plurality of first and second pulleys (41, 42), as shown in FIG. 2, a uniform force can be applied over the entire width direction of the left portion (22) and the right portion (23), so that force can be stably applied simultaneously to the upper and lower parts of the wearer's abdomen. The arrangement and coupling method of the plurality of first pulleys (41) and the first wire (51) shown in FIG. 2 are merely a preferred embodiment and can be changed as desired depending on a specific application environment or design.

**In** another non-limiting embodiment, the at least one wire (50) may include only a single wire (50). In this case, as shown in FIG. 1, it is preferable that the plurality of first pulleys (41) adjacent to the actuator (60) be configured as an even number, and that the plurality of second pulleys (42) distant from the actuator (60) also be configured as an odd number. The single wire (50) may form a closed loop by being connected from the actuator (60) to the plurality of first pulleys (41), to the back portion (21), and to the plurality of second pulleys (42), and then, via the back portion (21) again, connected to the actuator (60).

More specifically, with reference to FIG. 1, the wire (50) extending from the actuator (60) is first connected to the plurality of first pulleys (41). The wire (50) is wound sequentially and alternately, in the same manner as described above, around the pulleys among the plurality of first pulleys (41) that are fixed to the back-portion (21) side fixed portion (34) (first, third, fifth, and seventh pulleys) and the pulleys fixed to the left-portion (22) side fixed portion (34) (second, fourth, sixth, and eighth pulleys). The wire (50) extending from the last, eighth pulley among the plurality of first pulleys (41) does not become fixed to the back-portion (21) side fixed portion (34) but extends in the longitudinal direction passing through the back portion (21) and then reaches the second pulleys (42). The wire (50) that has extended to the second pulleys (42) is then connected sequentially in reverse order-from a ninth pulley to a first pulley-among the plurality of second pulleys (42), and the wire (50) extending from the first pulley among the plurality of second pulleys (42) again extends in the longitudinal direction passing through the back portion (21), reaches the actuator (60), and is fixed to a wire pulley of the actuator (60). Thus, the single wire (50) included in this embodiment forms a closed loop connected in the order of actuator (60) → plurality of first pulleys (41) → back portion (21) → plurality of second pulleys (42) → back portion (21) → actuator (60). By using only a single wire (50) forming such a closed loop, there is an advantage that both the first pulleys (41) of the first stretchable portion (31) and the second pulleys (42) of the second stretchable portion (32) can be driven simultaneously by a single actuator (60).

## Actuator

The belt-type dynamic orthosis (10) according to one aspect of the present invention includes an actuator (60) disposed in either one of the left portion (22) and the right portion (23) and configured to wind or unwind at least one wire (50). The actuator (60) may be controlled by the controller (80). In the present invention, since it is sufficient that the actuator (60) perform only the function of unwinding or pulling the wire (50), the specific configuration of the actuator (60) is not particularly limited.

In a non-limiting embodiment, the actuator (60) includes, as a power source, a motor, preferably a brushless DC motor (BLDC motor), and more preferably an electronically commutated (EC) motor, and may be configured to use a worm gear and/or a worm wheel as a speed reducer to amplify the wire driving force and to miniaturize and lighten the drive unit. A wire pulley capable of unwinding and pulling the wire (50) is disposed at an output end of the actuator (60) thus configured, and the wire (50) is ultimately driven by controlling rotation of the wire pulley.

The belt-type dynamic orthosis (10) according to one aspect of the present invention is a device that the wearer must wear in daily life. Therefore, the actuator must inevitably be driven wirelessly and is preferably designed to be driven at a low voltage. In a non-limiting embodiment, a low-voltage power supply may be applied to the actuator through the controller.

In another non-limiting embodiment, the actuator (60) may be a pneumatic actuator or a hydraulic actuator.

## Load Cell

The belt-type dynamic orthosis (10) according to one aspect of the present invention may include at least one load cell (70). The load cell (70) is a type of electronic device used to measure the weight of an object or a force; in the present invention, it is used to measure a tension applied to the wire (50) at the first pulley (41) or the second pulley (42) when the wire (50) is pulled and released. The load cell (70) itself may employ conventional techniques, and a detailed description and illustration of the structure and operating method of the load cell (70) will be omitted.

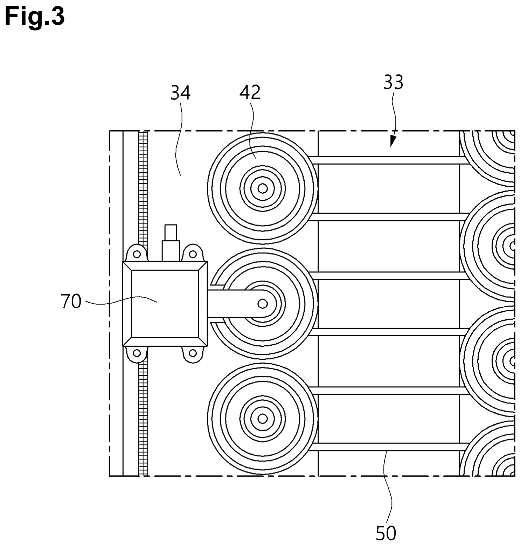

The load cell (70) may be connected to any one pulley among the plurality of first pulleys (41), or to any one pulley among the plurality of second pulleys (42). That is, as shown in FIG. 3, the load cell (70) may be connected to any one pulley in a middle of the multi-pulley mechanism (40) of the first stretchable portion (31) or the second stretchable portion (32). It is sufficient that the load cell (70) be connected to only one of the first pulley (41) and the second pulley (42); it need not necessarily be connected to a pulley at a specific position, nor is it necessary that it be connected to pulleys on both the first pulley (41) and the second pulley (42) sides.

When the wire (50) is pulled or released by the wire pulley of the actuator (60), the load cell (70) is configured to measure a tension applied to the wire (50) through the connected pulley. The tension applied to the wire (50) measured by the load cell (70) is sent to the controller (80), and the controller (80) may estimate the wearer's intra-abdominal pressure by inputting the measured tension applied to the wire (50) into pressure-tension modeling.

## Inertial Measurement Sensor

The belt-type dynamic orthosis (10) according to one aspect of the present invention may include at least two inertial measurement sensors (90) (inertial measurement units, IMU sensors). The inertial measurement sensor (90) is an electronic sensor used to measure and analyze the motion of an object; in the present invention, the wearer's posture may be estimated by using at least two inertial measurement sensors (90). The communication method between the controller (80) and the inertial measurement sensor (90) is not particularly limited; however, when connected by wire, there may be restrictions on movement while wearing, causing discomfort to the wearer, and therefore it is preferable that the controller (80) and the inertial measurement sensor (90) be configured to communicate with each other wirelessly.

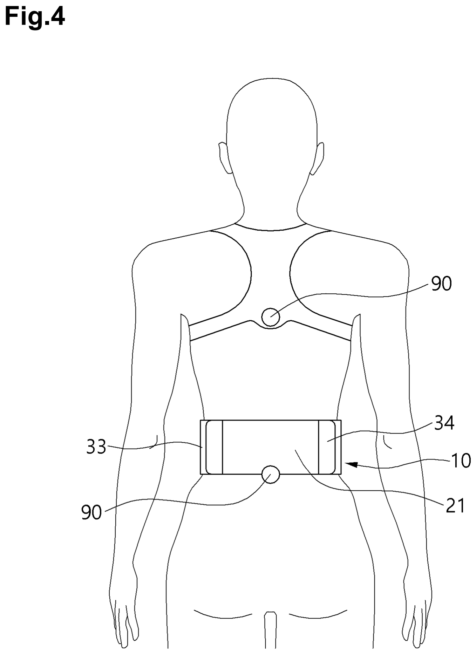

In a non-limiting embodiment, the belt-type dynamic orthosis (10) according to one aspect of the present invention may include two inertial measurement sensors (90). As shown in FIG. 4, it is preferable that the two inertial measurement sensors (90) be positioned so as to be disposed, when worn, at positions corresponding to the wearer's fifth thoracic vertebra (T5) and second sacral vertebra (S2), respectively, on the back. The two inertial measurement sensors (90) may be provided simultaneously in a single unit, or, as shown in FIG. 4, may be provided in two separate units (for example, a shoulder belt and a belt).

When the wearer changes posture, such as by bending or straightening the waist, a relative angle or a relative position of the two inertial measurement sensors (90) disposed at positions corresponding to T5 and S2 changes. The controller (80) may track the wearer's spinal movements by measuring the change in the relative angle or the relative position of the two inertial measurement sensors (90), and thereby estimate the wearer's posture.

## Controller

The belt-type dynamic orthosis (10) according to one aspect of the present invention includes a controller (80) for controlling the actuator (60). As shown in FIG. 1, the controller (80) may be built into the actuator (60), or may be provided as a unit separate from the actuator (60).

The controller (80) may be programmed to estimate the wearer's intra-abdominal pressure by using the load cell (70) and pre-stored pressure-tension modeling.

In a non-limiting embodiment, estimation of intra-abdominal pressure by the pressure-tension modeling may follow a method of measuring, via the load cell, a tension of the wire applied to a pulley of the belt-type dynamic orthosis, and estimating a pressure applied to the abdomen through the Young-Laplace law. At this time, the Young-Laplace law is applied to a fluid interface and formulates a correlation between a pressure difference between fluids on both sides of the interface and a tension of a membrane.

$$P_\alpha - P_\beta = \frac{2F_t}{R} \qquad (1)$$

Here, $P_\alpha$ and $P_\beta$ denote internal and external pressures, respectively; R denotes a radius of curvature; and Ft denotes a membrane tension. Assuming the human torso is cylindrical and transforming and applying the equation, the following expression can be

$$P_{abd} = \frac{K \cdot n \cdot F_t}{L_{ac} \cdot L_{width}} \qquad (2)$$

obtained:

Here, $P_{abd}$ denotes a pressure applied to the abdomen, i.e., intra-abdominal pressure. K is a constant value for conversion into units used in medicine; n is the number of layers of the belt; Ft is the tension; Lac is the wearer's abdominal circumference; and Lwidth denotes the height length of the belt.

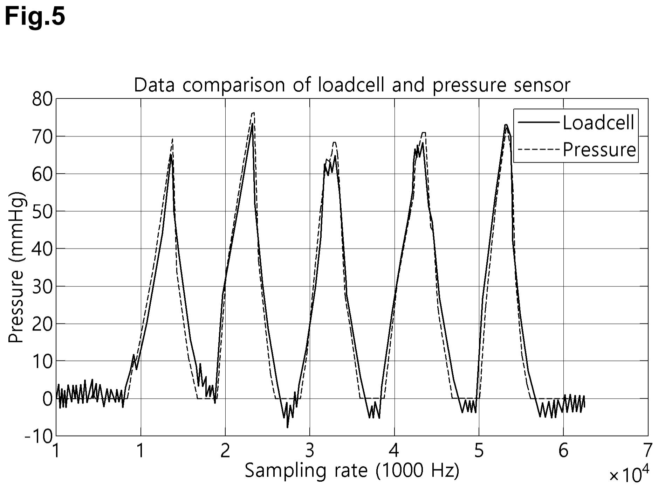

Through the above equation, the intra-abdominal pressure applied to the wearer can be estimated, and the results of estimation as compared with a commercial pressure sensor are as shown in FIG. 5 (RMSE of about 4 mmHg).

FIG. 5 shows a pressure measurement value according to the intra-abdominal pressure estimation technique mounted on the belt-type dynamic orthosis according to one embodiment of the present invention and a comparative pressure value of a commercial pressure sensor (blue: proposed device, red: commercial sensor).

More specifically, the controller (80) may receive, from the load cell (70), a measured value of a tension applied to the wire (50) passing through the first pulley (41) or the second pulley (42), and may estimate the wearer's intra-abdominal pressure by substituting the received tension measurement value into pre-stored pressure-tension modeling. The pre-stored pressure-tension modeling is initially set based on the wearer's abdominal circumference at the time of first wearing, and accordingly may be set differently in a customized manner for each wearer. The controller (80), based on the estimated intra-abdominal pressure, controls the actuator (60)-more specifically, controls rotation of a wire pulley disposed at an output end of the actuator (60)-to adjust the tension applied to the wire (50), thereby dynamically providing optimal abdominal compression suitable for the wearer's current intra-abdominal pressure.

The wire tension applied to the pulley is measured via the load cell, and the pressure applied to the abdomen can be estimated through the Young-Laplace law. The Young-Laplace law is applied to a fluid interface and formulates a correlation between a pressure difference between fluids on both sides of the interface and a tension of a membrane.

In a non-limiting embodiment, in addition to the wearer's intra-abdominal pressure estimated by using the load cell (70), the controller (80) may be configured to control the actuator (60) further in consideration of the wearer's posture. Specifically, as described above, the belt-type dynamic orthosis (10) according to one aspect of the present invention may include at least two inertial measurement sensors (90), preferably two inertial measurement sensors (90) positioned so as to be disposed, when worn, at positions corresponding to the wearer's fifth thoracic vertebra (T5) and second sacral vertebra (S2) on the back. In this embodiment, the controller (80) further includes a pressure profile according to the wearer's posture. Preferably, in this embodiment, the controller (80) further includes a pressure profile according to a relative angle of the two inertial measurement sensors (90). First, the controller (80) calculates a relative angle of the two inertial measurement sensors (90) to estimate the wearer's posture. Then, so that the intra-abdominal pressure estimated by using the load cell (70) and the pressure-tension modeling dynamically follows the pressure profile according to the estimated posture of the wearer, the controller controls rotation of the wire pulley disposed at the output end of the actuator (60) to adjust the tension applied to the wire (50), thereby dynamically providing optimal abdominal compression suitable for the wearer's intra-abdominal pressure and posture.

Meanwhile, the controller (80) may include an interface for input and output of data. By using the interface of the controller (80), an initial value of the wearer's abdominal circumference, the pressure profile, the pressure-tension modeling, and the like can be input into the controller (80). In addition, through the interface, the controller (80) may be connected to an external device such as a computer to initialize the orthosis or the inertial sensors, or to check a history of changes in the wearer's intra-abdominal pressure recorded in the controller (80).

## Wearing Method

Next, a method of wearing the belt-type dynamic orthosis (10) according to one aspect of the present invention will be described in detail.

First, the belt-type dynamic orthosis (10) according to one aspect of the present invention is prepared, and the wearer's abdominal circumference is measured before wearing the belt-type dynamic orthosis (10). The controller (80) is connected to a device such as a computer through the interface, and initial values of the belt-type dynamic orthosis (10) are adjusted based on the abdominal circumference. For example, an initial length of the wire (50) may be adjusted to fit the abdominal circumference, and a pressure profile or pressure-tension modeling suitable for the abdominal circumference may also be input.

Next, the wearer wears the belt-type dynamic orthosis (10). As shown in FIG. 4, the wearer places the belt-type dynamic orthosis (10) against the back and adjusts the positions so that the two inertial measurement sensors (90) are respectively located at predetermined positions, i.e., at positions corresponding to the fifth thoracic vertebra (T5) and the second sacral vertebra (S2); then wraps the left portion (22) and the first stretchable portion (31) around the left flank and wraps the right portion (23) and the second stretchable portion (32) around the right flank to encircle the waist as a whole; and finally fastens engagement means (24) such as Velcro provided on the right portion (23) and the left portion (22) to complete wearing.

After wearing, the wearer stands upright and straightens the waist, and then initializes the inertial measurement sensors (90). Then, through the controller (80), driving of the actuator (60) is commanded to start operation of the belt-type dynamic orthosis (10), and the belt-type dynamic orthosis (10) provides an appropriate compressive force by controlling the actuator (60) according to a lumbar angle measured by the wearer's inertial measurement sensors (90) when there is flexion or extension of the lumbar spine.

The belt-type dynamic orthosis according to the present invention does not drive the actuator when it recognizes that the lumbar angle is 0 degrees while the wearer is standing; however, when it recognizes that the waist is bent at a certain angle, the actuator keeps operating continuously to apply a target intra-abdominal pressure value. Therefore, if the device is to be temporarily put on or taken off, the device should be stopped for a while before proceeding.

In the above-described embodiment, the belt-type dynamic orthosis adjusts the widths of the first and second stretchable portions by using the actuator, pulleys, and wire; however, it is also possible without limitation to use other types of drivers. For example, in a non-limiting embodiment, appropriate abdominal pressure may be provided by adjusting the width of the stretchable portions by a pneumatic actuator or a hydraulic actuator.

## Embodiment(s)

The inventors manufactured a belt-type dynamic orthosis according to the above-described configuration and conducted an experiment on five healthy male subjects. Specifically, for the bare case (without any belt), the belt case (wearing a commercial belt), and the robot case (wearing the belt-type dynamic orthosis according to the present invention), the activation of muscles around the lumbar spine was compared when the same lumbar motion was performed in each case.

The male subjects who participated in the experiment lifted a 5 kg box, lowered the upper body to an angle at which the box reached the floor (the lower body being fixed), and then lifted it back to an upright posture; this motion was repeatedly performed five times at one cycle per three seconds.

In this experiment, a total of six surface electromyography sensors were attached to the right and left sides of the rectus abdominis, the external oblique, and the erector spinae to confirm muscle activity. In addition, a linear acceleration sensor was attached to the fifth thoracic vertebra to measure fore-aft sway of the upper body.

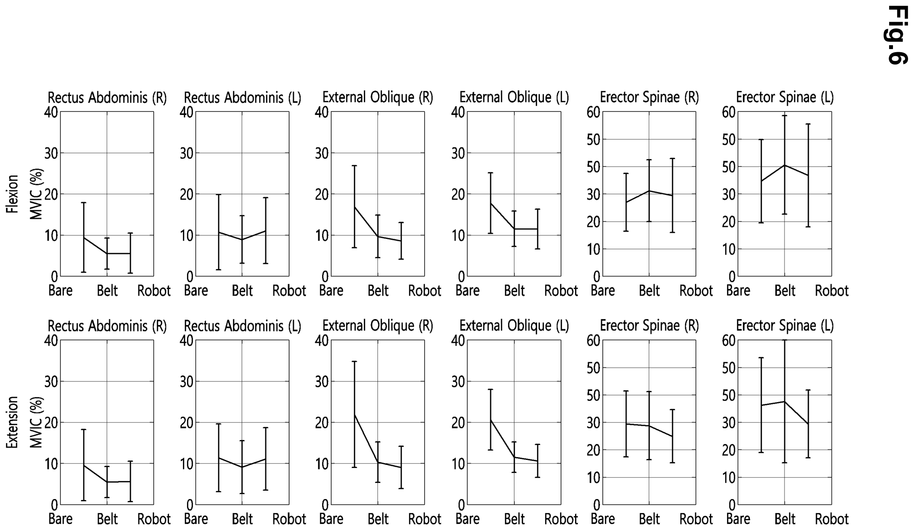

FIG. 6 is a graph showing, in an experiment on five male subjects, results of measuring muscle activity during a flexion section (with a 5 kg load box while the waist is bent) and an extension section (while the waist is straightened).

FIG. 6 shows the muscle activity results measured by six surface electromyography sensors according to the subjects' postures in the above experiment. The upper graph relates to the flexion section, and the lower graph relates to the extension section; in each graph, the Y-axis indicates muscle activity (100% indicating maximum muscle contraction). The indicated values are mean $\pm$ standard deviation of the muscle activity measured by each sensor.

Referring to the experimental results of FIG. 6, it can be confirmed that, for the right rectus abdominis (Rectus abdominis R) and the left and right external oblique (External oblique L/R), when a commercial belt or the belt-type dynamic orthosis according to the present invention is worn, muscle activity is reduced compared to the bare case, which means that the burden on the muscles is reduced. In addition, in some muscles, it can be confirmed that wearing the belt-type dynamic orthosis according to the present invention results in further reduction in muscle activity compared to wearing the commercial belt.

Referring to the results for the left and right erector spinae (Erector spinae L/R) in FIG. 6, when the commercial belt is worn, its static characteristics act as resistance against the direction of motion in the flexion and extension sections of the waist, whereby muscle activity increases relative to the bare case. However, when the belt-type dynamic orthosis according to the present invention is worn, because dynamic intra-abdominal pressure and support are provided according to spinal motion, resistance decreases while support increases, and as a result, lower muscle activity than in the bare case or when wearing the commercial belt-that is, a reduction in the burden on spinal muscles-can be confirmed.

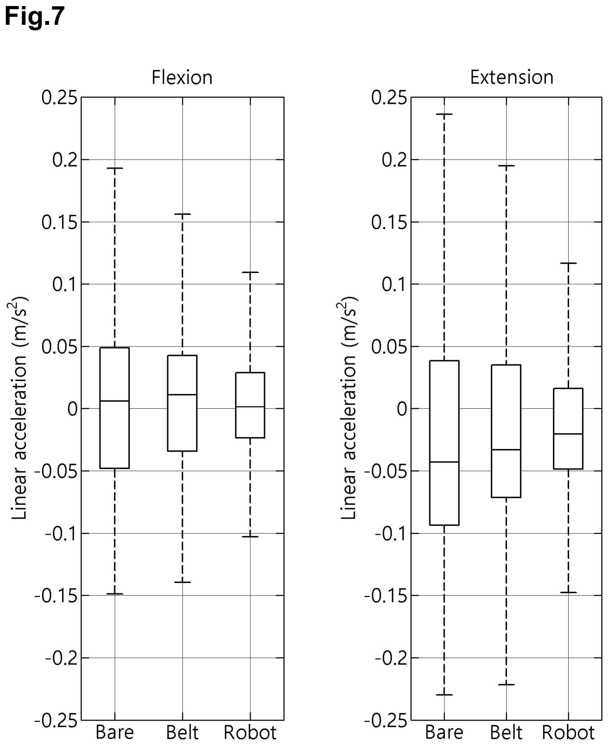

FIG. 7 is a graph showing, in an experiment on five male subjects, results of measuring linear acceleration in a fore-aft swaying direction of the torso to confirm the effect of spinal stability.

Meanwhile, FIG. 7 shows, by means of an inertial sensor attached to the fifth thoracic vertebra, results of measuring linear acceleration in the fore-aft swaying direction of the torso, i.e., the degree of vibration. In the graph of FIG. 7, yellow indicates the bare case, orange indicates the case of wearing a commercial belt, and red indicates the case of wearing the belt-type dynamic orthosis according to the present invention, each as a box plot.

Referring to the experimental results of FIG. 7, when the belt-type dynamic orthosis according to the present invention is worn, the range of the box plot is the smallest, from which it can be confirmed that, in the movement of the experiment, fore-aft vibration of the upper body is reduced by the belt-type dynamic orthosis according to the present invention, thereby increasing spinal stability.

The above description is merely illustrative of the technical idea of the present invention, and those of ordinary skill in the art to which the present invention pertains will be able to make various changes and modifications without departing from the essential characteristics of the present invention. Accordingly, the embodiments disclosed herein are not intended to limit but to explain the technical idea of the present invention, and the scope of the technical idea of the present invention is not limited by these embodiments. The scope of protection of the present invention shall be construed according to the claims below, and all technical ideas within an equivalent scope thereof shall be construed as being included within the scope of rights of the present invention.

**[Description of Reference Numerals]**

10: Belt-type dynamic orthosis
20: Belt body
21: Back portion
22: Left portion
23: Right portion
24: Engagement means
30: Stretchable portion
31: First stretchable portion
32: Second stretchable portion
33: Stretchable part
34: Fixed portion
40: Multi-pulley mechanism
41: First pulley
42: Second pulley
50: Wire
51: First wire
52: Second wire
60: Actuator
70: Load cell
80: Controller
90: Inertial measurement sensor

**Claims**

1. A belt-type dynamic orthosis comprising:

a belt body including a back portion that faces a wearer's back and left and right portions respectively located to the left and right of the back portion and configured to be mutually couplable;
a first stretchable portion located between the back portion and the left portion;
a second stretchable portion located between the back portion and the right portion;
a plurality of first pulleys disposed in the first stretchable portion;
a plurality of second pulleys disposed in the second stretchable portion;
at least one wire connected to the plurality of first pulleys and the plurality of second pulleys;
an actuator disposed in either one of the left portion and the right portion and configured to wind or unwind the at least one wire;
at least one load cell connected to any one pulley among the first pulleys or to any one pulley among the second pulleys, the at least one load cell being configured to measure a tension applied to the wire when the at least one wire is pulled and released; and

a controller configured to control the actuator.

2. The belt-type dynamic orthosis of claim 1, wherein

pressure-tension modeling representing a relationship between a wearer's intra-abdominal pressure and a tension applied to the wire is stored in the controller, and
the controller is configured to estimate the wearer's intra-abdominal pressure by inputting a tension measured by the load cell into the prestored pressure-tension modeling and, based on the estimated intra-abdominal pressure, control the actuator to adjust the tension applied to the wire.

3. The belt-type dynamic orthosis of claim 2, wherein

the belt-type dynamic orthosis further comprises two inertial measurement sensors respectively disposed at a back region and a waist region of the wearer, and
a pressure profile according to the wearer's posture is stored in the controller, and
the controller is configured to calculate a relative angle of the two inertial measurement sensors to estimate the wearer's posture and to control the actuator so that the estimated intra-abdominal pressure dynamically follows the pressure profile according to the estimated posture, thereby adjusting the tension applied to the wire.

4. The belt-type dynamic orthosis of claim 3, wherein

the back region is a position corresponding to a fifth thoracic vertebra (T5), and
the waist region is a position corresponding to a second sacral vertebra (S2).

5. The belt-type dynamic orthosis of any one of claims 1 to 3, wherein
the load cell is connected to any one pulley among the plurality of first pulleys or to any one pulley among the plurality of second pulleys.

6. The belt-type dynamic orthosis of any one of claims 1 to 3, wherein

the plurality of first pulleys are alternately disposed on a back-side fixed portion of the first stretchable portion and on a left-side fixed portion of the first stretchable portion, and
the plurality of second pulleys are alternately disposed on a back-side fixed portion of the second stretchable portion and on a right-side fixed portion of the second stretchable portion.

7. The belt-type dynamic orthosis of any one of claims 1 to 3, wherein

the plurality of first pulleys and the plurality of second pulleys are each an even number,
the at least one wire comprises a first wire connected to the plurality of first pulleys and a second wire connected to the plurality of second pulleys,
one end of the first wire is connected to the actuator and the other end is fixed to the back-side fixed portion of the first stretchable portion, and
one end of the second wire is connected to the actuator and the other end is fixed to the back-side fixed portion of the second stretchable portion.

8. The belt-type dynamic orthosis of any one of claims 1 to 3, wherein

the plurality of first pulleys and the plurality of second pulleys are each an odd number,
the at least one wire comprises a first wire connected to the plurality of first pulleys and a second wire connected to the plurality of second pulleys,
one end of the first wire is connected to the actuator and the other end is fixed to a left-side fixed portion of the first stretchable portion, and
one end of the second wire is connected to the actuator and the other end is fixed to a right-side fixed portion of the second stretchable portion.

9. The belt-type dynamic orthosis of any one of claims 1 to 3, wherein

the at least one wire consists of a single wire,
pulleys among the plurality of first pulleys and the plurality of second pulleys that are adjacent to the actuator are configured as an even number, and pulleys among the plurality of first pulleys and the plurality of second pulleys that are distant from the actuator are configured as an odd number, and
the single wire forms a closed loop by being connected from the actuator to the plurality of first pulleys, to the back portion, and to the plurality of second pulleys and then, via the back portion again, to the actuator.

10. The belt-type dynamic orthosis of any one of claims 1 to 3, wherein

the first stretchable portion is positioned to face a wearer's left flank, and
the second stretchable portion is positioned to face the wearer's right flank.

**11.** The belt-type dynamic orthosis of any one of claims 1 to 3, wherein
the actuator comprises a brushless DC motor, a worm gear, and a wire pulley.

**12.** The belt-type dynamic orthosis of any one of claims 1 to 3, wherein
the actuator is a pneumatic actuator or a hydraulic actuator.

# Fig. 1.

# Fig.2

# Fig.3

# Fig.4

# Fig.5

**Fig.6**

**Fig.7**

## INTERNATIONAL SEARCH REPORT

| International application No. |
| --- |
| **PCT/KR2023/011090** |

| | |
| --- | --- |
| **A. CLASSIFICATION OF SUBJECT MATTER** | |
| **A61F 5/02**(2006.01)i; **A61F 5/30**(2006.01)i | |
| According to International Patent Classification (IPC) or to both national classification and IPC | |

**B. FIELDS SEARCHED**

Minimum documentation searched (classification system followed by classification symbols)

A61F 5/02(2006.01); A41D 13/05(2006.01); A61F 2/68(2006.01); A61F 5/03(2006.01); A61G 7/10(2006.01); A61N 1/04(2006.01); A61N 1/32(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 척추(spine), 요추(lumbar), 동적(dynamic), 복대(abdominal belt), 신축성(stretchiness), 로드셀(load cell), 풀리(pulley), 와이어(wire), 장력(tension), 측정(measurement), 복압(abdominal pressure)

**C. DOCUMENTS CONSIDERED TO BE RELEVANT**

| Category* | Citation of document, with indication, where appropriate, of the relevant passages | Relevant to claim No. |
| --- | --- | --- |
| A | KR 10-2378441 B1 (PARK, Eui Han) 23 March 2022 (2022-03-23)<br>See paragraphs [0007], [0020]-[0022] and [0030]-[0041]; claims 1, 4 and 6; and figures 1-4. | 1-12 |
| A | KR 10-2015-0118683 A (LEE, Seong Jong) 23 October 2015 (2015-10-23)<br>See paragraph [0001]; claims 1-3; and figures 1 and 3. | 1-12 |
| A | KR 10-1523846 B1 (INJE UNIVERSITY INDUSTRY-ACADEMIC COOPERATION FOUNDATION) 28 May 2015 (2015-05-28)<br>See paragraphs [0011] and [0036]; and claims 1 and 3-4. | 1-12 |
| A | WO 2012-109524 A1 (OSSUR HF et al.) 16 August 2012 (2012-08-16)<br>See entire document. | 1-12 |
| A | JP 2012-213543 A (TOYOTA MOTOR CORP.) 08 November 2012 (2012-11-08)<br>See entire document. | 1-12 |

☑ Further documents are listed in the continuation of Box C.    ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **11 January 2024** | **12 January 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| | International application No. |
| | **PCT/KR2023/011090** |

### C. DOCUMENTS CONSIDERED TO BE RELEVANT

| Category* | Citation of document, with indication, where appropriate, of the relevant passages | Relevant to claim No. |
|---|---|---|
| A | KR 10-1664351 B1 (LEE, Jae Jin) 11 October 2016 (2016-10-11)<br>    See entire document. | 1-12 |
| PX | 김주완 등. 요부 안정화를 위한 복대형 입는 로봇 개발. 로봇학회 논문지. June 2023, vol. 18, no. 2, pp. 189-196 (KIM, Joo Wan et al. Development of Brace-type Wearable Robot for Lumbar Stabilization. KRS Journal.)<br>    See abstract; pages 189-193; and figures 3, 4 and 7. | 1-12 |

Form PCT/ISA/210 (second sheet) (July 2022)

## INTERNATIONAL SEARCH REPORT
### Information on patent family members

International application No.

**PCT/KR2023/011090**

| Patent document cited in search report | | | Publication date (day/month/year) | Patent family member(s) | | | Publication date (day/month/year) |
|---|---|---|---|---|---|---|---|
| KR | 10-2378441 | B1 | 23 March 2022 | KR | 10-2021-0123486 | A | 14 October 2021 |
| KR | 10-2015-0118683 | A | 23 October 2015 | None | | | |
| KR | 10-1523846 | B1 | 28 May 2015 | KR | 10-2015-0049710 | A | 08 May 2015 |
| WO | 2012-109524 | A1 | 16 August 2012 | CN | 102333502 | A | 25 January 2012 |
| | | | | CN | 102333502 | B | 25 June 2014 |
| | | | | CN | 102695479 | A | 26 September 2012 |
| | | | | CN | 102695479 | B | 24 December 2014 |
| | | | | CN | 103442669 | A | 11 December 2013 |
| | | | | CN | 103442669 | B | 16 September 2015 |
| | | | | CN | 104068954 | A | 01 October 2014 |
| | | | | CN | 104068954 | B | 19 September 2017 |
| | | | | EP | 2400936 | A1 | 04 January 2012 |
| | | | | EP | 2400936 | B1 | 09 September 2015 |
| | | | | EP | 2496191 | A1 | 12 September 2012 |
| | | | | EP | 2496191 | B1 | 02 July 2014 |
| | | | | EP | 2672937 | A1 | 18 December 2013 |
| | | | | EP | 2672937 | B1 | 19 April 2017 |
| | | | | US | 10264835 | B2 | 23 April 2019 |
| | | | | US | 10617552 | B2 | 14 April 2020 |
| | | | | US | 10828186 | B2 | 10 November 2020 |
| | | | | US | 2010-0217167 | A1 | 26 August 2010 |
| | | | | US | 2011-0105971 | A1 | 05 May 2011 |
| | | | | US | 2012-0022419 | A1 | 26 January 2012 |
| | | | | US | 2012-0204381 | A1 | 16 August 2012 |
| | | | | US | 2013-0006158 | A1 | 03 January 2013 |
| | | | | US | 2014-0155798 | A1 | 05 June 2014 |
| | | | | US | 2014-0200497 | A1 | 17 July 2014 |
| | | | | US | 2015-0121657 | A1 | 07 May 2015 |
| | | | | US | 2015-0141892 | A1 | 21 May 2015 |
| | | | | US | 2016-0074200 | A1 | 17 March 2016 |
| | | | | US | 2016-0324678 | A1 | 10 November 2016 |
| | | | | US | 2017-0189220 | A1 | 06 July 2017 |
| | | | | US | 2021-0077289 | A1 | 18 March 2021 |
| | | | | US | 8172779 | B2 | 08 May 2012 |
| | | | | US | 8303528 | B2 | 06 November 2012 |
| | | | | US | 8657769 | B2 | 25 February 2014 |
| | | | | US | 8926537 | B2 | 06 January 2015 |
| | | | | US | 8939925 | B2 | 27 January 2015 |
| | | | | US | 8945034 | B2 | 03 February 2015 |
| | | | | US | 9220625 | B2 | 29 December 2015 |
| | | | | US | 9414953 | B2 | 16 August 2016 |
| | | | | US | 9597219 | B2 | 21 March 2017 |
| | | | | WO | 2010-098880 | A1 | 02 September 2010 |
| | | | | WO | 2010-098880 | A4 | 11 November 2010 |
| | | | | WO | 2011-056217 | A1 | 12 May 2011 |
| JP | 2012-213543 | A | 08 November 2012 | None | | | |
| KR | 10-1664351 | B1 | 11 October 2016 | None | | | |

Form PCT/ISA/210 (patent family annex) (July 2022)

## EP 4 699 582 A1

**Patent documents cited in the description**

- KR 101664351